# EUROPEAN PATENT APPLICATION

(11) **EP 4 782 045 A1**
(43) Date of publication of application: **29.07.2026**
(21) Application number: 25305119.7
(22) Date of filing: 28.01.2025
(51) Int. Cl.: A61M 25/00, A61M 25/01

(54) **CATHETER WITH A DOUBLE CURVATURE**

(71) Applicant: Digisurge, 34170 Castelnau-Le-Lez (FR)
(72) Inventor: ROMOSCANU, Alexandre Ioan, 1207 Geneva (CH)
(74) Representative: Tranvouez, Edern Morgan

(57) **Abstract**

The invention relates to a catheter (1) comprising a proximal part (2) that can be passively curved and a distal part (4) comprising a first portion (5) able to be actively steered according to a first curvature and at least one second portion (6) able to be actively steered according to a second curvature, independent from the first curvature.

## Description

### Technical domain

The invention concerns a catheter with a double curvature.

### Prior art

Endovascular delivery systems, such as catheters, are known to be used in various procedures to deliver medical devices or instruments to a target location inside a patient's body that is not readily accessible by surgery or where access without surgery is desirable. A catheter, as described herein, can be used to deliver a medical device, such as a stent, a heart valve, a graft, a clip, a repair device, a valve treatment device, etc., to a location in a patient's body.

Access to a target location inside the patient's body, including but not limited to, a blood vessel, an esophagus, a trachea, any portion of the gastrointestinal tract, a lymphatic vessel, to name a few. Catheters are known in the art and have been commonly used to reach target locations inside a patient's body.

A steerable catheter is also known which includes a handle, a body of the catheter and a steering mechanism. The catheter's body extends from a proximal end attached to the handle to a distal end. The body comprises a rigid or flexible proximal part which can be passively curved and a flexible distal part which can be steered according to a curvature, using the steering mechanism. The steering mechanism is attached to the handle and is configured to steer the flexible distal part. In some embodiments, the steering mechanism includes a steering element, a control member and actuations elements, such as pullwires and compression coils. The actuation elements extend from proximal end attached to the steering element to distal end attached to flexible distal part of the catheter. Actuating the control member of the steering mechanism moves the steering element to increase or release tension in one of the actuation elements.

In some embodiments, the actuation elements are eccentrically positioned to cause the body to curve in a determined direction. A catheter can also comprise an axially non compressive sleeve extending co-axially over at least a portion of the actuation element or pullwire.

The steering of the distal part in a desired direction, according to a curvature, is interesting to better follow the curvature of the vessel in which the catheter is inserted. It is also interesting to position and/or deploy a device, such as an implant, in a given position in a body cavity, such as, but not limited to, one of the four heart's chambers.

In all the prior art implementations, only a single segment of the catheter is steerable according to only one curvature. However, it appears that generally, a vessel comprises several curvatures, in two or three dimensions. Also, a given position in a body cavity, such as, but not limited to, one of the four heart's chambers, may be more conveniently reached with a catheter featuring more than one curvature.

### Abstract

The invention aims to solve this problem by providing a catheter steerable along at least two different curvatures. Said curvatures can be non-coplanar or coplanar.

The object of the invention is a catheter comprising a proximal part that can be passively curved and a distal part comprising a first portion able to be actively steered according to a first curvature and at least one second portion able to be actively steered according to a second curvature, independent from the first curvature, wherein
- the distal part comprises a hypotube arranged to resist axial compression but to allow flexion, whose length defines the length of the distal part,
- the first portion comprises
   - at least one proximal compression coil,
   - for each said at least one proximal compression coil, an associated proximal stop located along the distal part,
   - for each said at least one proximal compression coil, an associated proximal actuation pullwire,
   - for each proximal actuation pullwire, an associated medial stop located along the distal part, more distal than the associated proximal stop,
   - each at least one proximal compression coil running from a handle located at a proximal end of the proximal part to stop at its associated proximal stop,
   - each associated proximal actuation pullwire running from the handle located at the proximal end of the proximal part, through its associated proximal compression coil and affixed to its associated medial stop, so as to allow a flexural actuation of the hypotube along the first portion running from the associated proximal stop to the associated medial stop, when the associated proximal actuation pullwire is pulled with respect to its associated proximal compression coil and
- the second portion comprises
   - at least one distal compression coil,
   - for each said at least one distal compression coil, an associated medial stop located along the distal part,
   - for each said at least one distal compression coil, an associated distal actuation pullwire,
   - for each distal actuation pullwire, an associated distal stop located along the distal part, more distal than the associated medial stop,
   - each at least one distal compression coil running from the handle located at the proximal end of the proximal part to stop at its associated medial stop,
   - each associated distal actuation pullwire running from the handle located at the proximal end of the proximal part, through its associated distal compression coil and affixed to its associated distal stop, so as to allow a flexural actuation of the hypotube along the second portion running from the associated medial stop to the associated distal stop, when the associated distal actuation pullwire is pulled with respect to its associated distal compression coil.

Some features or particular embodiments, usable alone or in combination, are :
- a compression coil presents a compressive stiffness greater than the compressive stiffness of a hypotube, outside of the neutral plane of said hypotube,
- the hypotube comprises a proximal hypotube and a distal hypotube, and all proximal stops are located at the proximal end of the proximal hypotube, all medial stops are located at the distal end of the proximal hypotube and all distal stops are located at the distal end of the distal hypotube,
- the proximal stop, the medial stop, the at least one proximal compression coil and the at least one proximal actuation pullwire are housed at least partially inside the proximal hypotube, and the medial stop, the distal stop, the at least one distal compression coil and the at least one distal actuation pullwire are housed at least partially inside the distal hypotube,

- the catheter further comprises a supporting part able to guide, radially and angularly, at least all along the length of hypotubes, at least one of said at least one proximal compression coil or at least one of said at least one distal compression coil, and preferably all compression coils,
- the supporting part comprises a helicoidal part, rolled up along the catheter's axis, and for each proximal compression coil or distal compression coil, a series of axially aligned first axial holes, axially drilled in every spire, each series of first holes being able to welcome a compression coil, wherein the section of the helicoidal part is flat along the longitudinal/axial direction and generally square,
- the supporting part comprises a first flexible tube, aligned with the catheter's axis, comprising, for each proximal compression coil or distal compression coil, an axial groove, located on its inner face, able to welcome a compression coil, and a first inner central coil, aligned with the catheter's axis, tangent to the welcomed compression coils, so as to maintain the compression coils in axial grooves,
- the supporting part comprises a second flexible tube, aligned with the catheter's axis, comprising, for each proximal compression coil or distal compression coil, an axial tunnel, able to welcome a compression coil,
- the second flexible tube is made by inserting an inner tube into an outer tube of a smaller diameter, the folds in the inner tube forming the axial tunnels,
- the supporting part comprises a plurality of support rings, preferentially equidistant, each support ring comprising, for each proximal compression coil or distal compression coil, a second axial hole able to welcome a compression coil, and a second inner central coil, tangent to the compression coils,
- all proximal stops are housed in a proximal stop ring and/or all medial stops are housed in a medial stop ring and/or all distal stops are housed in a distal stop ring, preferentially integral to one of the hypotube,
- the at least one proximal compression coil and the at least one proximal actuation pullwire are equiangularly spaced, and the at least one distal compression coil and the at least one distal actuation pullwire are equiangularly spaced, intercalated with respect to the proximal compression coils /actuation pullwires,
- the at least one proximal compression coil comprises four proximal compression coils and the at least one proximal actuation pullwire comprises four proximal actuation pullwires, and the at least one distal compression coil comprises four distal compression coils and the at least one distal actuation pullwire comprises four distal actuation pullwires,
- a stop ring comprises, periodically alternated on its perimeter, at least one longitudinal hole able to welcome and to stop a compression coil or to stop an actuation pullwire and a longitudinal groove able to freely welcome or to stop a compression coil, the total number of longitudinal holes and longitudinal grooves being equal to the total number of compression coils.

### Drawings

The invention would be better understood by the reading of the following detailed description, only provided as an example, and with respect to the annexed figures in which:
[Fig. 1] shows, in perspective view, a catheter,
[Fig. 2] shows, in perspective view, the catheter of Figure 1 with the hypotube removed,
[Fig. 3] shows, in cut perspective, the catheter of Figure 1,
[Fig. 4] shows, in perspective view, a stop ring,
[Fig. 5] shows, in cut perspective view, a hypotube with integral stops,
[Fig. 6] shows, in perspective view, a zoomed detail of the hypotube,
[Fig. 7] shows, in perspective view, a first embodiment of a support part,
[Fig. 8] shows, in perspective view, a second embodiment of a support part,
[Fig. 9] shows, in perspective view, a detail of Figure 8,
[Fig. 10] shows, in perspective view, a third embodiment of a support part,
[Fig. 11] shows, in sectional view, a detail of Figure 10,
[Fig. 12] shows, in perspective view, a fourth embodiment of a support part.

### Detailled description

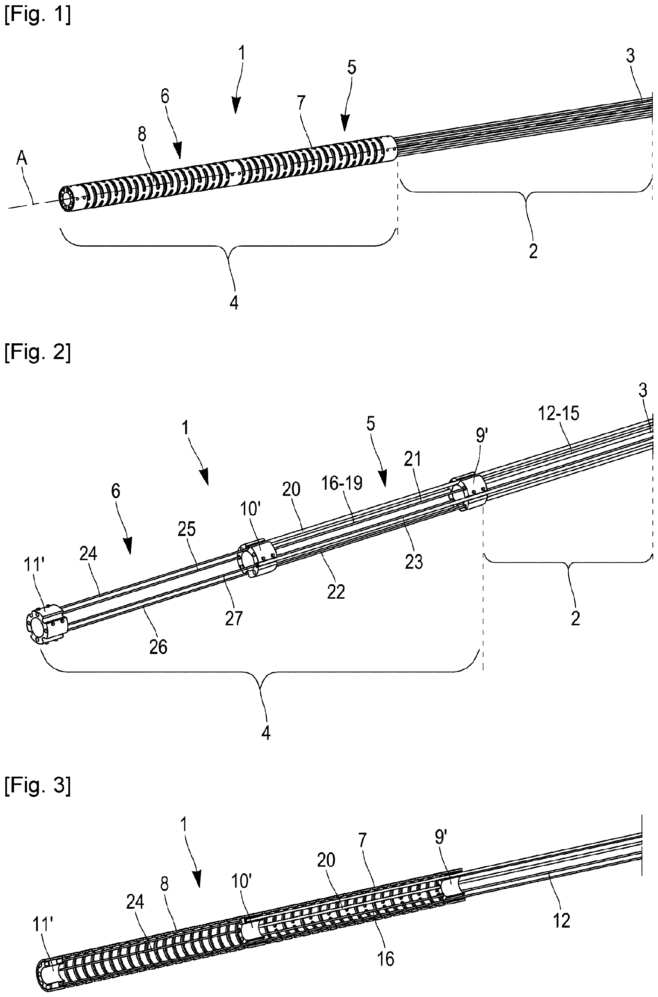

With respect to Figures 1-3, the invention concerns a catheter 1. Said catheter 1 comprises a proximal part 2 and a distal part 4. The distal part 4 is the part which is firstly introduced into a patient's vessel. The proximal part 2, at its proximal end 3 comprises a handle (not illustrated) from where the catheter 1 is handled and tilted, with respect to curvatures.

The proximal part 2 can be passively curved, e.g. when a vessel turns, the proximal part 4 follows the curvature of the vessel.

The distal part 4 comprises a first portion 5 and at least a second portion 6. The fist portion can be actively steered according to a first curvature. Said at least one second portion 6 can to be actively steered according to a second curvature. The second curvature is independent from the first curvature.

The distal part 4 comprises a hypotube 7, 8. A hypotube is a tube adapted to resist axial compression but to allow flexion. The length of said hypotube 7, 8 runs all along the length of the distal part 4.

The first portion 5 comprises at least one proximal compression coil 12-15. For each said at least one proximal compression coil 12-15, there is an associated proximal stop 9 located along the distal part 4. For each said at least one proximal compression coil 12-15, there is an associated proximal actuation pullwire 16-19. For each proximal actuation pullwire 16-19, there is an associated medial stop 10 located along the distal part 4. The medial stop 10 is more distal than the associated proximal stop 9. Each at least one proximal compression coil 12-15 runs from a handle located at a proximal end 3 of the proximal part 2. Each at least one proximal compression coil 12-15 stops at its associated proximal stop 9. Each associated proximal actuation pullwire 16-19 runs from the handle located at the proximal end 3 of the proximal part 2. It runs through its associated proximal compression coil 12-15, in which it is inserted. Each associated proximal actuation pullwire 16-19 is affixed to its associated medial stop 10, so as to allow a flexural actuation of the hypotube 7, 8 along the first portion 5 running from the associated proximal stop 9 to the associated medial stop 10, when the associated proximal actuation pullwire 16-19 is pulled with respect to its associated proximal compression coil 12-15, at the handle.

Since a proximal compression coil 12-15 is considered to be non-compressible along its axis, up to the material's elastic modulus, a pull applied to the associated proximal pullwire 16-19 shortens the length of the proximal pullwire 16-19 between the proximal stop 9 where the proximal compressive coil 12-15 stops and the medial stop 10 where the proximal pullwire 16-19 is affixed. Following this shortening, beneficially not applied along the catheter's axis A, thanks to a peripheral location of the coils/pullwires, the hypotube 7, 8, which is also considered to be non-compressible, has no other option than to flex between the proximal stop 9 and the medial stop 10.

The second portion 6 comprises at least one distal compression coil 20-23. For each said at least one distal compression coil 20-23, there is an associated medial stop 10 located along the distal part 4. For each said at least one distal compression coil 20-23, there is an associated distal actuation pullwire 24-27. For each distal actuation pullwire 24-27, there is an associated distal stop 11 located along the distal part 4. The distal stop 11 is more distal than the associated medial stop 10. Each at least one distal compression coil 20-23 runs from the handle located at the proximal end 3 of the proximal part 2. Each at least one distal compression coil 20-23 stops at its associated medial stop 10. Each associated distal actuation pullwire 24-27 runs from the handle located at the proximal end 3 of the proximal part 2. It runs through its associated distal compression coil 20-23, in which it is inserted. Each associated distal actuation pullwire 24-27 is affixed to its associated distal stop 11, so as to allow a flexural actuation of the hypotube 7, 8 along the second portion 6 running from the associated medial stop 10 to the associated distal stop 11, when the associated distal actuation pullwire 24-27 is pulled with respect to its associated distal compression coil 20-23.

Since a distal compression coil 20-23 is considered to be non-compressible along its axis, a pull applied to the associated distal pullwire 24-27, shortens the length of the distal pullwire 24-27 between the medial stop 10 where the distal compressive coil 20-23 stops and the distal stop 11 where the distal pullwire 24-27 is affixed. Following this shortening, beneficially not applied along the catheter's axis A, thanks to a peripheral location of the coils/pullwires, the hypotube 7, 8, which is also considered to be non-compressible, has no other option than to flex between the medial stop 10 and the distal stop 11.

Since said at least one proximal actuation pullwire 16-19 is separated from said at least one distal actuation pullwire 24-27, the flexural actuation of the first position 5 is independent from the flexural actuation of the second portion 6.

Since the distal actuation pullwires 24-27 do not exert any bending moment on the proximal first portion 5, said portion 5 will not be flexurally loaded upon actuation of distal actuation pullwires 24-27, resulting in the possibility to steer the distal second portion 6 without steering the proximal first portion 5.

According to another feature, compression coil 12-15, 20-23, be it a proximal one or a distal one, presents a compressive stiffness greater than the compressive stiffness of a hypotube 7, 8, outside of the neutral plane of said hypotube 7, 8. This allows a hypotube to flex when a pullwire 16-19, 24-27 is pulled with respect to its associated compression coil 12-15, 20-23.

According to another feature, the hypotube 7, 8 is preferentially composed of a proximal hypotube 7 and a distal hypotube 8. Preferentially, both proximal hypotube 7 and distal hypotube are of the same length.

As previously described, a stop 9, 10, 11 can be located all along the distal part 4, at any place. The only condition is that the proximal stop 9 is more proximal than the medial stop 10, which in turn is more proximal than the distal stop 11. So doing, each line, comprising a compression coil and its associated pullwire, can define a first portion 5 on which it can apply a curvature, independently from the second portion 6. Each line is independent from the other lines. This can be useful to define a catheter 1 presenting dissymmetrical curvature capabilities, for specific applications.

Alternately, in order to manufacture a symmetrical catheter, whose actuation command is easier, all proximal stops 9, respectively all medial stops 10, respectively all distal stops 11 can be located at the same length along the catheter's axis A.

According to another feature, as illustrated at Figures, all proximal stops 9 are located at the proximal end of the proximal hypotube 7, all medial stops 10 are located at the distal end of the proximal hypotube 7, which is also the proximal end of the distal hypotube 8, and all distal stops 11 are located at the distal end of the distal hypotube 8.

According to another feature, all the lines are housed inside the hypotube 7, 8. So, the proximal stop 9, the medial stop 10, the at least one proximal compression coil 12-15 and the at least one proximal actuation pullwire 16-19 are housed at least partially inside the proximal hypotube 7. Said components form a proximal line. Said proximal line has its proximal compression coil 12-15 ending at the proximal end of the proximal hypotube 7 and the free range of the associated proximal pullwire 16-19 running all along the proximal hypotube 7 and thus is able to steer the flexion of said proximal hypotube 7.

Similarly, the medial stop 10, the distal stop 11, the at least one distal compression coil 20-23 and the at least one distal actuation pullwire 24-27 are housed at least partially inside the distal hypotube 8. Said components form a distal line. Said distal line has its distal compression coil 20-23 ending at the proximal end of the distal hypotube 8 and the free range of the associated distal pullwire 24-27 running all along the distal hypotube 8 and thus is able to steer the flexion of said distal hypotube 8.

Since the lines are housed inside the hypotube 7, 8, they can move inside the catheter 1. Such a move can crowd the central part of the catheter 1 that needs to stay clear e.g. to carry a tool or treatment from the handle to the distal end. Such a move can also modify, due to the flexural compliance or the compressive coil, the reduced length of a pullwire and thus modify the intended flexion of the hypotube 7, 8.

According to another feature, a catheter 1 preferentially further comprises a supporting part. Said supporting part is able to guide, radially and angularly, generally at least all along the length of hypotube 7, 8, at least one of said at least one proximal compression coil 12-15 or at least one of said at least one distal compression coil 20-23, and preferably all compression coils 12-15, 20-23. Optionally, said supporting part can be all along the length of the whole catheter 1. Such a supporting part can be made upon many embodiments. Four such embodiments are presented hereafter.

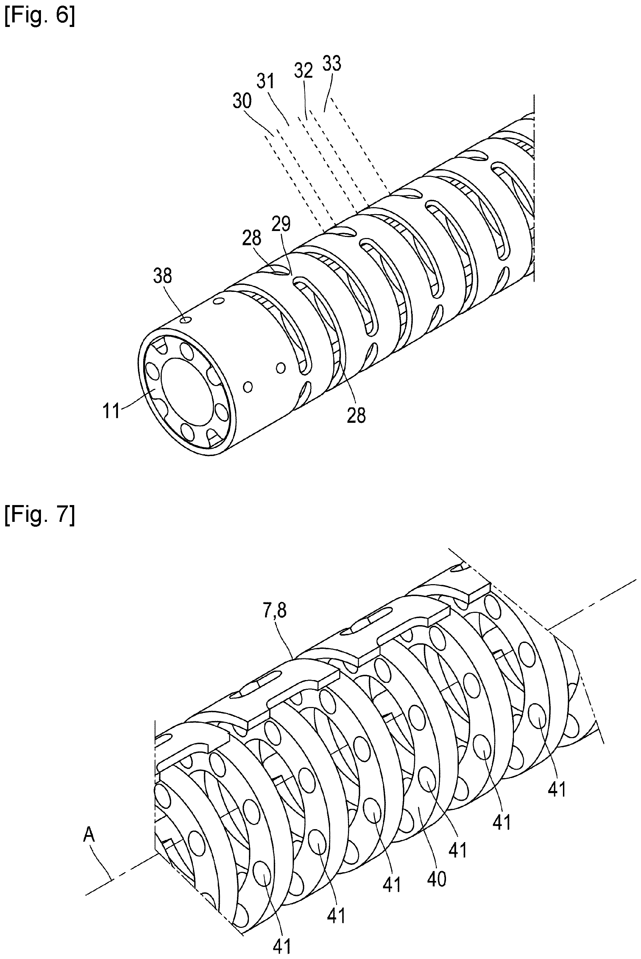

According to a first embodiment, illustrated at Figure 7, the supporting part comprises a helicoidal part 40. Said helicoidal part 40 is rolled up along the catheter's axis A to form a tube-shaped part. For each proximal compression coil 12-15 or distal compression coil 20-23, the helicoidal part 40 comprised a series of axially aligned first axial holes 41. All first holes 41 are axially drilled, along the catheter's axis A, in every spire. That is, in every spire, there is as many first hole 41 as compression coil 12-15, 20-23. Each axial series of first holes 41 is able to welcome one compression coil 12-15, 20-23.

Preferentially, the section of the helicoidal part 40 is flat along the longitudinal/axial direction A. More preferentially, said section is generally square. Said helicoidal part 40 is preferentially made of metallic material.

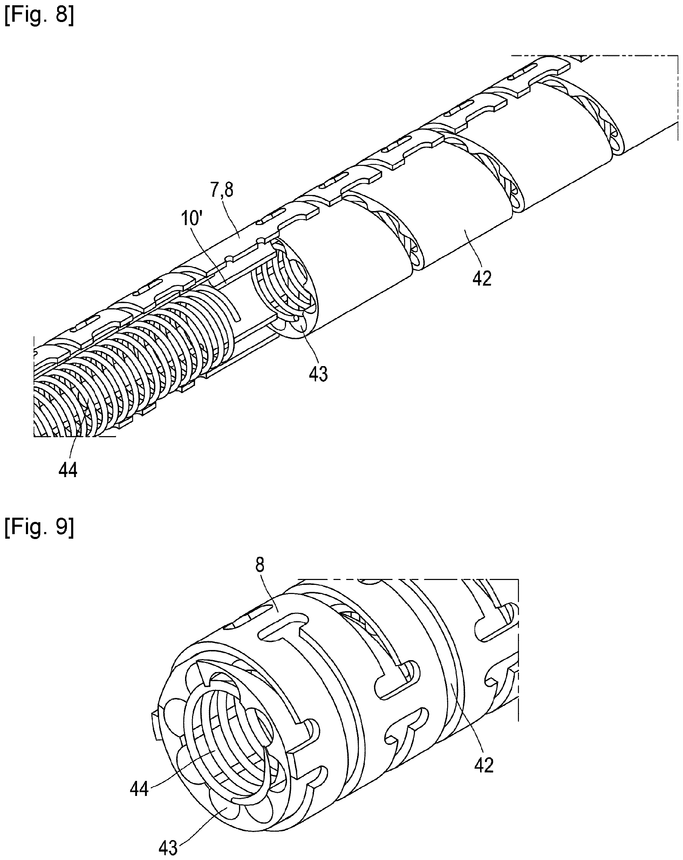

According to a second embodiment, illustrated at Figures 8-9, the supporting part comprises a first flexible tube 42. Said flexible tube 42 is aligned with the catheter's axis A and is flexible to allow the flexion of the hypotubes 7, 8. For each proximal compression coil 12-15 or distal compression coil 20-23, the flexible tube 42 comprises an axial groove 43. Said at least one groove 43, axially oriented along the catheter's axis A, is located on the inner face of the flexible tube 42. Each groove 43 is able to welcome one compression coil 12-15, 20-23. The groove 43 maintains the welcomed compression coil 12-15, 20-23 laterally and maintains its orientation along the axis A. The supporting part further comprises a first inner central coil 44. Said first inner coil 44 is central and aligned with the axis A, that is, it shares its axis with the catheter's axis A. The diameter of said first inner central coil 44 is determined so as to have the peripheral of the first inner central coil tangent to the compression coils 12-15, 20-23 welcomed in the grooves 43. So, the first central inner coil 44 maintains the compression coils 12-15, 20-23 in their respective axial grooves 43.

Said first flexible tube 42 can be continuous depending on its flexural stiffness. If it is not flexible enough, a spiral cut, as illustrated, can advantageously reduce said flexural stiffness. Said first flexible tube 42 can be made out of metallic or plastic material. Said first flexible tube 42 can be manufactured by extrusion.

The first flexible tube 42 could have a length shorter, as an example between 1 to 5 mm shorter, than the distance between proximal ring stop 9' and the median ring stop 10'.

As an alternative not shown, the first flexible tube 42 and the first inner central coil 44 could be made of a single component. Such a single component coulb be formed by a reinforced coil including the axial grooves 43. Such alternative is advantageous, since it requires less elements and so reduced the risk of failure.

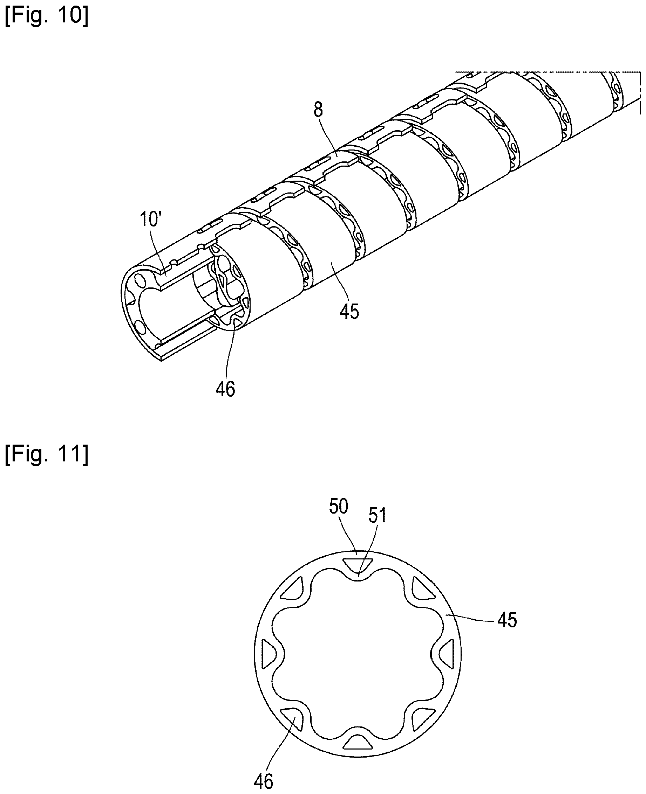

According to a third embodiment, illustrated at Figures 10-11, the supporting part comprises a second flexible tube 45. Said second flexible tube 45 is colinear to the catheter's axis A. It comprises, for each proximal compression coil 12-15 or distal compression coil 20-23, an axial tunnel 46, able to welcome a compression coil 12-15, 20-23.

According to another feature, the second flexible tube 45 is designed as if an inner tube 51 has been inserted into an outer tube 50 of a smaller diameter. The diameter difference creates folds in the inner tube 51, preferably equiangularly spaced. Each fold forms an axial tunnel 46.

Said second flexible tube 45 can be continuous depending on its flexural stiffness. If it is not flexible enough, a spiral cut, as illustrated, can advantageously reduce said flexural stiffness. Said second flexible tube 45 can be made out of metallic or plastic material. Said second flexible tube 45 can be manufactured by extrusion. The second flexible tube 45 could have a length shorter, as an example between 1 to 5 mm shorter, than the distance between proximal ring stop 9' and the median ring stop 10'.

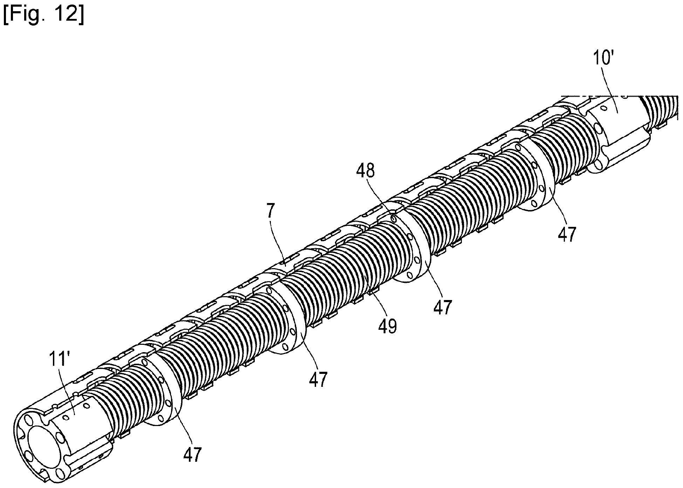

According to a fourth embodiment, illustrated at Figure 12, the supporting part comprises a plurality of support rings 47, disposed along axis A, preferentially equidistantly. Each support ring 47 comprises, for each proximal compression coil 12-15 or distal compression coil 20-23, a second axial hole 48, drilled parallel to the axis A, able to welcome a compression coil 12-15, 20-23. Support rings 47 are all angularly indexed so as to align the second axial holes 48 belonging to the same compression coil 12-15, 20-23.

The supporting part further comprises a second inner central coil 49, tangent to the compression coils 12-15, 20-23. Said second inner central coil 49 is similar, in every feature, to the first inner central coil 44, and serves the same purposes.

The first inner central coil 44 could be made of a flat wire, two consecutive turns of the coil being in contact through the flat s of the wire.

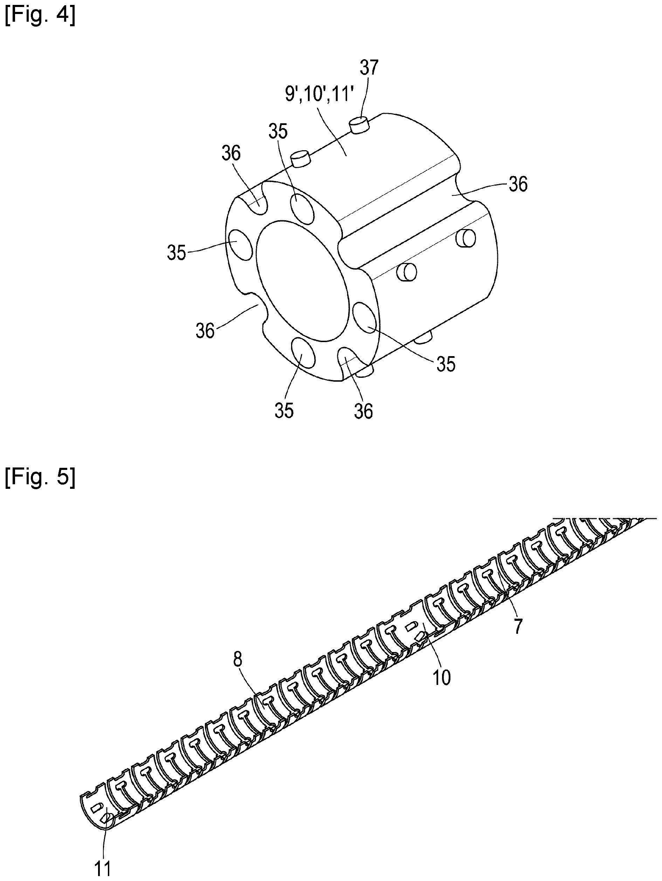

According to another feature, more particularly illustrated at Figure 4, all proximal stops 9 are housed in a proximal stop ring 9' and/or all medial stops 10 are housed in a medial stop ring 10' and/or all distal stops 11 are housed in a distal stop ring 11'. Said stop rings 9', 10', 11', thanks to a smart design, fits all the three, proximal, medial and distal positions, with the same exact part.
Said stop ring 9', 10', 11' can be inserted in the hypotube 7, 8. A stop ring 9', 10', 11' comprises tenons 37 able to insert in corresponding holes 38 managed in the hypotubes 7, 8, so as to block the stop ring 9', 10', 11' both axially and angularly.

Alternately a stop ring 9', 10', 11' can be made integral to the hypotube 7, 8.

With reference to Figure 5, is shown medial stops 10 and distal stops 11, integral to the hypotube 7, 8. It must be noticed that such an embodiment can be applied to ring stops 9', 10', 11', with all stops 9, 10 ,11 at the same length (symmetrical embodiment) or to stops 9, 10, 11 located at any length (non-symmetrical embodiment).

According to another feature, the proximal hypotube 7 and the distal hypotube 8 can be made integral, that is, in one single component.

As is known in prior art, a hypotube has to be stiff in compression and compliant in flexion.

This can be obtained by assembling rigid articulated links to make a hypotube (embodiment not illustrated).

As illustrated at Figure 6, this can be obtained by drilling a tube. Said drilling can be e.g. according to a radially cut pattern. Such a pattern can comprise cuts 28 whose main extension is in a transverse section of the tube.

According to another feature, as illustrated at Figure 6, the hypotube 7, 8 can be obtained from a tube, preferably metallic, machined with a preferentially periodic pattern. Said pattern can comprise four axial sections of noticeably the same axial length. A first section 30 is mainly holed, that is, holes 28, for instance two of them here, occupy the main portion of the peripheral, with only two small diametrically opposed links 29 remaining. A link 29 is conformed to exhibit a relatively high flexural compliance and a relatively low axial compliance. A second section 31 is full, in that it contains matter on the whole peripheral. The second section 31 acts as a separator. A third section 32 is similar to the first section but rotated 90° around the axis A of the tube. So doing the links 29 are not aligned from section 1 to section 3, but equiangularly interspersed. A fourth section 33 is full, similar to the second section 31, to provide a separator. Said four sections pattern is periodically repeated all along the axis A.

According to another feature, as illustrated e.g. at Figure 5 or 9, the shape of holes 28 may vary.

According to another feature, the at least one proximal compression coil 12-15 and the at least one proximal actuation pullwire 16-19 are equiangularly spaced. This allows a better prediction of the angles of flexion obtained in function of the length of the pullwires pulled.

Similarly, the at least one distal compression coil 20-23 and the at least one distal actuation pullwire 24-27 are also equiangularly spaced. Preferentially, they are interspersed with respect to the proximal compression coils /actuation pullwires.

According to a first embodiment, they are regularly interspersed.

Alternately, they are interspersed so that the distal ones are closest to the proximal as possible. The smallest angular shift is then determined by the respective diameters of the compression coils 12-15, 20-23.

According to another feature, as illustrated at Figures, the at least one proximal compression coil 12-15 comprises four proximal compression coils and the at least one proximal actuation pullwire 16-19 comprises four proximal actuation pullwires. Such a number of four allows a rather good angular resolution in flexion of the proximal hypotube 7 by combining tractions of at least one and at most two adjacent of said proximal actuation pullwire 16-19.

Simmilarly, the at least one distal compression coil 20-23 comprises four distal compression coils and the at least one distal actuation pullwire 24-27 comprises four distal actuation pullwires. Such a number of four allows a rather good angular resolution in flexion of the distal hypotube 8 by combining tractions of at least one and at most two adjacent of said distal actuation pullwire 24-27. Since it is the same number as the proximal ones, they nicely intersperse.

According to another feature, more particularly illustrated at Figure 4, a stop ring 9', 10', 11' comprises, periodically alternated on its perimeter, at least one longitudinal hole 35 and as many longitudinal groove 36. Such an axial / longitudinal hole 35 can be used either to welcome and to stop an end of a compression coil 12-15, 20-23 or to stop an end of an actuation pullwire 16-19, 24-27, by locking it in said axial / longitudinal hole 35. Such an axial / longitudinal groove 36 can be used to freely welcome or to stop a compression coil 12-15, 20-23. Freely welcome, here means to guide angularly and radially, while allowing a translation along the groove's axis. The total number of longitudinal holes 35 and longitudinal grooves 36 is equal to the total number of compression coils 12-15, 20-23.

The invention has been illustrated and described in detail in the drawings and the previous description. This description must be considered as being illustrative and given as an example and not as limiting the scope of the invention to this description alone. Numerous variations are possible.

### Reference signs list

A: axis,
1: catheter,
2: proximal part,
3: proximal end,
4: distal part,
5: first portion,
6: second portion,
7: proximal hypotube,
8: distal hypotube,
9: proximal stop,
9': proximal ring stop,
10: medial stop,
10': medial ring stop,
11: distal stop,
11': distal ring stop,
12-15: proximal compression coil,
16-19: proximal actuation pullwire,
20-23: distal compression coil,
24-27: distal actuation pullwire,
28: cut,
29: link,
30-33: section,
35: longitudinal hole,
36: longitudinal groove,
37: tenon,
38: tenon's hole,
40: helicoidal part,
41: first hole,
42: first flexible tube,
43: axial groove,
44: first central inner coil,
45: second flexible tube,
46: axial tunnel,
47: support ring,
48: second hole,
49: second inner central coil,
50: outer tube,
51: inner tube.

## Claims

1. Catheter (1) comprising a proximal part (2) that can be passively curved and a distal part (4) comprising a first portion (5) able to be actively steered according to a first curvature and at least one second portion (6) able to be actively steered according to a second curvature, independent from the first curvature, ***characterized in that***
- the distal part (4) comprises a hypotube (7, 8) arranged to resist axial compression but to allow flexion, whose length defines the length of the distal part (4),
- the first portion (5) comprises
- at least one proximal compression coil (12-15),
- for each said at least one proximal compression coil (12-15), an associated proximal stop (9) located along the distal part (4),
- for each said at least one proximal compression coil (12-15), an associated proximal actuation pullwire (16-19),
- for each proximal actuation pullwire (16-19), an associated medial stop (10) located along the distal part (4), more distal than the associated proximal stop (9),
- each at least one proximal compression coil (12-15) running from a handle located at a proximal end (3) of the proximal part (2) to stop at its associated proximal stop (9),
- each associated proximal actuation pullwire (16-19) running from the handle located at the proximal end (3) of the proximal part (2), through its associated proximal compression coil (12-15) and affixed to its associated medial stop (10), so as to allow a flexural actuation of the hypotube (7, 8) along the first portion (5) running from the associated proximal stop (9) to the associated medial stop (10), when the associated proximal actuation pullwire (16-19) is pulled with respect to its associated proximal compression coil (12-15) and
- the second portion (6) comprises
- at least one distal compression coil (20-23),
- for each said at least one distal compression coil (20-23), an associated medial stop (10) located along the distal part (4),
- for each said at least one distal compression coil (20-23), an associated distal actuation pullwire (24-27),
- for each distal actuation pullwire (24-27), an associated distal stop (11) located along the distal part (4), more distal than the associated medial stop (10),
- each at least one distal compression coil (20-23) running from the handle located at the proximal end (3) of the proximal part (2) to stop at its associated medial stop (10),
- each associated distal actuation pullwire (24-27) running from the handle located at the proximal end (3) of the proximal part (2), through its associated distal compression coil (20-23) and affixed to its associated distal stop (11), so as to allow a flexural actuation of the hypotube (7, 8) along the second portion (6) running from the associated medial stop (10) to the associated distal stop (11), when the associated distal actuation pullwire (24-27) is pulled with respect to its associated distal compression coil (20-23).

2. Catheter (1) according to claim 1, wherein a compression coil (12-15, 20-23) presents a compressive stiffness greater than the compressive stiffness of a hypotube (7, 8), outside of the neutral plane of said hypotube (7, 8).

3. Catheter (1) according to anyone of claims 1 or 2, wherein the hypotube (7, 8) comprises a proximal hypotube (7) and a distal hypotube (8), and wherein all proximal stops (9) are located at the proximal end of the proximal hypotube (7), all medial stops (10) are located at the distal end of the proximal hypotube (7) and all distal stops (11) are located at the distal end of the distal hypotube (8).

4. Catheter (1) according to any one of claims 1 to 3, wherein the proximal stop (9), the medial stop (10), the at least one proximal compression coil (12-15) and the at least one proximal actuation pullwire (16-19) are housed at least partially inside the proximal hypotube (7), and the medial stop (10), the distal stop (11), the at least one distal compression coil (20-23) and the at least one distal actuation pullwire (24-27) are housed at least partially inside the distal hypotube (8).

5. Catheter (1) according to any one of claims 1 to 4, further comprising a supporting part able to guide, radially and angularly, at least all along the length of hypotubes (7, 8), at least one of said at least one proximal compression coil (12-15) or at least one of said at least one distal compression coil (20-23), and preferably all compression coils (12-15, 20-23).

6. Catheter (1) according to claim 5, wherein the supporting part comprises a helicoidal part (40), rolled up along the catheter's axis (A), and for each proximal compression coil (12-15) or distal compression coil (20-23), a series of axially aligned first axial holes (41), axially drilled in every spire, each series of first holes (41) being able to welcome a compression coil (12-15, 20-23), wherein the section of the helicoidal part (40) is flat along the longitudinal/axial direction and generally square.

7. Catheter (1) according to claim 5, wherein the supporting part comprises a first flexible tube (42), aligned with the catheter's axis (A), comprising, for each proximal compression coil (12-15) or distal compression coil (20-23), an axial groove (43), located on its inner face, able to welcome a compression coil (12-15, 20-23), and a first inner central coil (44), aligned with the catheter's axis (A), tangent to the welcomed compression coils (12-15, 20-23), so as to maintain the compression coils (12-15, 20-23) in axial grooves (43).

8. Catheter (1) according to claim 5, wherein the supporting part comprises a second flexible tube (45), aligned with the catheter's axis (A), comprising, for each proximal compression coil (12-15) or distal compression coil (20-23), an axial tunnel (46), able to welcome a compression coil (12-15, 20-23).

9. Catheter (1) according to claim 8, wherein the second flexible tube (45) is made by inserting an inner tube (51) into an outer tube (50) of a smaller diameter, the folds in the inner tube (51) forming the axial tunnels (46).

10. Catheter (1) according to claim 5, wherein the supporting part comprises a plurality of support rings (47), preferentially equidistant, each support ring (47) comprising, for each proximal compression coil (12-15) or distal compression coil (20-23), a second axial hole (48) able to welcome a compression coil (12-15, 20-23), and a second inner central coil (49), tangent to the compression coils (12-15, 20-23).

11. Catheter (1) according to any one of claims 3 to 4, wherein all proximal stops (9) are housed in a proximal stop ring (9') and/or all medial stops (10) are housed in a medial stop ring (10') and/or all distal stops (11) are housed in a distal stop ring (11'), preferentially integral to one of the hypotube (7, 8).

12. Catheter (1) according to anyone of claims 3 to 7, wherein the at least one proximal compression coil (12-15) and the at least one proximal actuation pullwire (16-19) are equiangularly spaced, and the at least one distal compression coil (20-23) and the at least one distal actuation pullwire (24-27) are equiangularly spaced, intercalated with respect to the proximal compression coils /actuation pullwires.

13. Catheter (1) according to anyone of claims 3 to 8, wherein the at least one proximal compression coil (12-15) comprises four proximal compression coils and the at least one proximal actuation pullwire (16-19) comprises four proximal actuation pullwires, and the at least one distal compression coil (20-23) comprises four distal compression coils and the at least one distal actuation pullwire (24-27) comprises four distal actuation pullwires.

14. Catheter (1) according to anyone of claims 3 to 9, wherein a stop ring (9', 10', 11') comprises, periodically alternated on its perimeter, at least one longitudinal hole (35) able to welcome and to stop a compression coil (12-15, 20-23) or to stop an actuation pullwire (16-19, 24-27) and a longitudinal groove (36) able to freely welcome or to stop a compression coil (12-15, 20-23), the total number of longitudinal holes (35) and longitudinal grooves (36) being equal to the total number of compression coils (12-15, 20-23).
